# EUROPEAN PATENT SPECIFICATION

(11) **EP 3 675 761 B1**
(45) Date of publication and mention of the grant of the patent: **11.02.2026**
(21) Application number: 18816290.3
(22) Date of filing: 14.09.2018
(51) Int. Cl.: A61B 18/14

(54) **DEVICES FOR REMODELING TISSUE**
VORRICHTUNGEN ZUR GEWEBEREMODELLIERUNG
DISPOSITIF DE REMODELAGE DE TISSU

(30) Priority: 14.09.2017 US 201762558565 P
(43) Date of publication of application: 08.07.2020
(73) Proprietor: Medtronic, Inc., Minneapolis, MN 55432 (US)
(72) Inventor: GIFFORD, Hanson S., III, Menlo Park, CA 94025 (US); MCLEAN, Matthew, Menlo Park, CA 94025 (US); KRISHNAMURTHY, Gaurav, Menlo Park, CA 94025 (US); FANN, James, Menlo Park, CA 94025 (US); SUTTON, Douglas, Menlo Park, CA 94025 (US)
(74) Representative: Zimmermann & Partner Patentanwälte mbB
(86) International application number: PCT/US2018/051211
(87) International publication number: WO 2020/055433

(56) References cited:
- EP-A1- 3 178 516
- EP-A1- 3 178 516
- WO-A1-2014/008489
- WO-A1-2014/008489
- WO-A2-2008/084244
- WO-A2-2008/084244
- US-A1- 2006 189 972
- US-A1- 2006 189 972
- US-A1- 2011 306 851
- US-A1- 2011 306 851
- US-A1- 2012 271 277

## Description

### FIELD OF THE PRESENT TECHNOLOGY

The present technology relates to RF devices used to remodel tissue. The device and methods disclosed herein have broad applicability to shrink collagenous tissue, and in particular they are well suited for remodeling cardiac tissue (e.g., a cardiac valve annulus and the chordae tendineae) to reduce regurgitation though the valve and enhance valve competency.

### BACKGROUND

Mitral annular dilatation is a common feature of mitral valve disease, especially in functional or secondary mitral valve disease. As the annulus dilates, the leaflets are pulled apart until the edges no longer coapt in systole resulting in regurgitation. Reducing the overall circumference of the annulus is one of the most common elements of successful surgical mitral valve repair. This can be surgically performed by sewing the mitral annulus to an annuloplasty ring having a smaller diameter than the annulus. This permanently reduces the mitral annular circumference, but it entails an open or minimally-invasive surgical procedure involving significant trauma, morbidity, and recovery time.

Many different catheter-based mitral annuloplasty concepts have been pursued. For example, devices have been placed in the coronary sinus paralleling the mitral annulus, or a number of anchors have been placed in the annulus and then pulled together.

Several techniques to perform mitral annuloplasty using radiofrequency (RF) energy have been attempted. For example, a ring of electrodes has been applied against the atrial surface of the annulus, and then RF energy is delivered between pairs of electrodes to heat and shrink the tissue. Another technique involves driving a pair of spaced-apart electrodes into the annular tissue and delivering RF energy between the electrodes to shrink the annular tissue.

Other techniques deliver RF energy via catheters to reshape tissue to perform other valve modifications, such as shrinking the length of chordae tendineae and shrinking heart valve leaflet tissue itself. However, these techniques have drawbacks, such as controlling the extent of shrinkage. For example, the mitral valve has delicate and carefully sculpted tissue features, which may need to be shrunk in only certain directions.

Chemically induced ablation has also been applied to the mitral valve. One such attempt is disclosed in the American Journal of Physiology and is entitled "Ablation of mitral annular and leaflet muscle: effects on annular and leaflet dynamics", Tomasz PubMed12969884. WO201/008489 A1 relates to devices for carotid body ablation, WO 2008/084244 A2 relates to devices for the treatment of diseased tissue, US 2011/306851 A1 relates to catheter for mapping and renal ablation, EP 3 178 516 A1 relates to stabilized spine electrophysiologic catheters, US 2006/189972 A1 relates to devices for uterine fibroid treatment and US 2012/271277 A1 relates to expandable catheter systems for peri-ostial injection and muscle and nerve fiber ablation.

Given the difficulties associated with current procedures, there remains the need for simple, effective, and less invasive devices and methods for treating dysfunctional heart valves.

### SUMMARY OF THE PRESENT TECHNCLOGY

The claimed subject-matter is defined by appended claim 1. Further embodiments are disclosed in the dependent claims. Aspects, embodiments and examples of the present disclosure which are not encompassed by the appended claims are not part of the claimed subject-matter and are provided for illustrative purposes. For example,the disclosed methods are not claimed as methods. An example method disclosed herein, is a minimally invasive method for reducing the size of a cardiac valve annulus in a beating heart, comprising:
a. advancing an energy delivery catheter into the heart proximate a cardiac valve annulus, the energy delivery catheter having at least two electrodes;
b. advancing the two electrodes such that the two electrodes pierce into the cardiac valve annulus at a distance from one another;
c. applying an approximating force to at least one of the two electrodes, thereby reducing the distance between the two electrodes; and
d. applying energy between the at least two electrodes, thereby heating and shrinking the annulus in a direction of the approximating force.

In the previous method, further comprising extending the two electrodes from the catheter by increasing a spacing between the two electrodes from a compact spacing to an extended spacing, wherein a spacing between the two electrodes in the extended spacing is greater than the spacing between the two electrodes in the compact spacing.

In any of the previous methods, the at least two electrodes may be configured to self-extend away from each other when unconstrained, and wherein increasing a spacing between the two electrodes includes allowing the two electrodes to self-extend away from each other.

In any of the previous methods, wherein increasing a spacing between the two electrodes includes inflating a bladder interposed between the two electrodes.

In any of the previous methods, wherein increasing a spacing between the two electrodes includes actuating a mechanism to actively increase the spacing between the two electrodes.

In any of the previous methods, the two electrodes include a first electrode and a second electrode, and the method includes:
a. withdrawing the first electrode from the annulus while leaving the second electrode embedded in the annulus;
b. pivoting the energy delivery catheter about the second electrode;
c. advancing the first electrode into the cardiac annulus;
d. applying an approximating force biasing at least one of the first or second electrodes toward the other; and
e. applying an energy between the first and second electrodes thereby heating and shrinking the annulus in a direction of the approximating force.

In any of the preceding methods, further comprising:
a. terminating delivery of the energy and allowing the valve annulus time to cool; and
b. removing the two electrodes from the annulus.

In any of the previous methods, wherein applying an approximating force includes advancing a sheath catheter toward the at least two electrodes.

In any of the previous methods, wherein applying an approximating force includes deflating the bladder between the electrodes.

In any of the previous methods, applying an approximating force includes actuating an approximating mechanism to actively decrease the spacing between the two electrodes.

Also disclosed is a minimally invasive method for selectively reducing the dimensions of a cardiac valve tissue in a beating heart, comprising:
a. advancing a delivery catheter into the heart, the delivery catheter having at least two engagement members and an energy delivery mechanism;
b. advancing the engagement members into the cardiac valve tissue such that engagement members are spaced apart from one another by a distance;
c. applying an approximating force to the engagement members; and
d. applying energy between the engagement members using the energy delivery member thereby shrinking the annulus cardiac tissue in a direction of the approximating force.

In the preceding method for selectively reducing the dimensions of cardiac tissue, further comprising extending the engagement members from the catheter by increasing a spacing between the engagement members from a compact spacing to an extended spacing, wherein the extended spacing is greater than the compact spacing.

In any of the preceding methods for selectively reducing the dimensions of cardiac tissue, wherein the engagement members are configured to self-extend away from each other when unconstrained, and wherein increasing a spacing between the engagement members includes allowing the engagement members to self-extend away from each other.

In any of the preceding methods for selectively reducing the dimensions of cardiac tissue, wherein increasing a spacing between the engagement members includes inflating a bladder interposed between the engagement members.

In any of the preceding methods for selectively reducing the dimensions of cardiac tissue, wherein increasing a spacing between the engagement members includes actuating an approximating mechanism to actively increase the spacing between the engagement members.

In any of the preceding methods for selectively reducing the dimensions of cardiac tissue, wherein the engagement members include a first engagement member and second engagement member, and the method further comprises:
a. withdrawing the first engagement member from the annulus cardiac tissue while leaving the second engagement member embedded in the cardiac tissue;
b. pivoting the energy delivery catheter about the second engagement member;
c. advancing the first engagement member into the cardiac tissue;
d. moving at least one of the engagement members toward the other along an approximating path; and
e. applying at least one of energy and chemical agent between the engagement members thereby shrinking the cardiac tissue annulus in the direction of the approximating path.

In any of the preceding methods for selectively reducing the dimensions of cardiac tissue, wherein moving at least one of the engagement members along an approximating path includes advancing the catheter toward the engagement members.

In any of the preceding methods for selectively reducing the dimensions of cardiac tissue, wherein moving at least one of the engagement members along an approximating path includes deflating the bladder between the engagement members.

In any of the preceding methods for selectively reducing the dimensions of cardiac tissue, wherein moving at least one of the engagement members along an approximating path includes actuating an approximating mechanism to actively decrease the spacing between the engagement members.

In any of the preceding methods, wherein applying energy includes applying an energy modality selected from the group of (bipolar, monopolar, resistive heating, ultrasound, laser, and microwave).

In any of the preceding methods for selectively reducing the dimensions of cardiac tissue, wherein the chemical agent is selected from the group of (phenol, and glutaraldehyde).

Also disclosed is a minimally invasive device for reducing the diameter of a cardiac valve annulus in a beating heart, comprising:
a. an elongate delivery catheter;
b. at least two engagement members carried by the delivery catheter, wherein the engagement members and catheter have a retracted position in which the engagement members are fully contained within the catheter and an extended position in which the engagement members extend beyond a distal end of the catheter;
c. a tissue shrinking component configured to deliver at least one of energy and a chemical agent between the two engagement members; and
d. an approximation mechanism configured to apply a force to the engagement members, wherein the force is selected from the group of an approximating force and a separating force.

In the above-described device, the tissue shrinking component comprises an energy delivery mechanism configured to deliver an energy modality selected from the group (bipolar, resistive heating, ultrasound, laser, and microwave).

In any of the above-described devices, the tissue shrinking component comprises a chemical agent is selected from the group of (phenol, and glutaraldehyde).

In any of the above-described devices, the tissue shrinking component is operably connected to the engagement members.

In any of the above-described devices, the approximation mechanism includes a linkage connecting the engagement members.

In the above-described device, the linkage may include a hinge.

In the above-described device, the approximation mechanism includes a pull-wire connected to the linkage such that pulling on the pull-wire applies an approximation force to the engagement members.

In any of the above-described devices, the approximation mechanism includes a sleeve surrounding at least a portion of the engagement members, and wherein advancing the sleeve biases the engagement members together.

### BRIEF DESCRIPTION OF THE DRAWINGS

FIG. 1 depicts an energy delivery device;
FIG. 2 depicts an energy delivery device;
FIG. 3 depicts an energy delivery device;
FIGs. 4A-4D depict an energy delivery device and a method for shrinking cardiac tissue in a selective direction;
FIGs. 5A-5D depict a method for shrinking cardiac tissue in a selective direction;
FIG. 6 depicts an energy delivery device;
FIGs. 7A-7C depict optional features of the energy delivery device of FIGs. 1-3; and
FIG. 8 depicts an energy delivery device.

### DETAILED DESCRIPTION

The present technology is useful for shrinking collagenous tissue in general, and it is particularly useful for shrinking cardiac tissue, such as the annulus of a cardiac valve and/or the chordae tendineae, in a controlled, predictable manner to reduce regurgitation through the valve.

### Annuloplasty

Several existing mitral annuloplasty techniques shrink collagen fibers by heating the fibers to a transition temperature. It is known that applying energy to heat collagenous tissue in a relaxed state causes it to shrink, and the shrinkage typically occurs in all directions. In general, the rate of shrinkage is greater in the direction of the fiber orientation. However, heating collagenous tissue while it is under a certain degree of tension often results in the collagen shrinking in dimensions other than the direction of the tension. This presents particular challenges for mitral valve procedures because the effect of ventricular pressure on the mitral annulus induces significant tension in the mitral annulus. The general stiffness of the mitral annulus and the tendency of the surrounding tissues, including muscular ventricular tissue, also tends to hold the collagen in its original shape even after applying energy. Moreover, the collagenous tissue in the annulus is surrounded by other tissue, such as muscle, which is not as likely to shrink when heated. As a result, existing mitral annuloplasty techniques may not shrink the collagen fibers in a desired manner.

The present technology is expected to overcome the drawbacks of existing mitral annuloplasty techniques by grasping the cardiac tissue and approximating if in the desired direction of shrinkage. Energy is applied to the tissue either during or after approximating the tissue. The desired shrinkage may be in a circumferential direction (e.g., around the cardiac valve annulus), or it may be in another direction. Approximating the tissue reduces the tension experienced by the cardiac tissue thereby preferentially shrinking the collagenous tissue in the desired direction. The force approximating the tissue may be maintained briefly after terminating energy delivery. The tissue will shrink further in the desired direction than it would without pre-approximation, and it will retain more of the shrinkage in the desired direction after the energy has been applied and the device is removed.

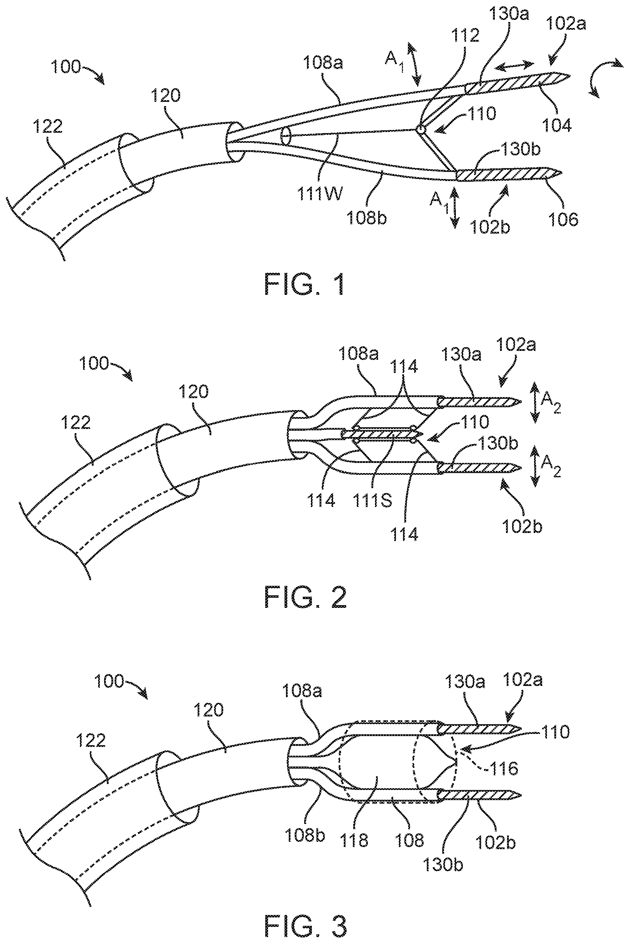

FIG. 1 depicts an energy delivery device 100 having a delivery catheter 120 and an optional guide catheter 122. The device 100 has a plurality of pin-shaped electrodes 102 (identified individually as a first electrode 102a and a second electrode 102b) at the distal end. The electrodes 102 can be independently advanced and/or retracted to insert them into and/or remove them from the annular tissue. The electrodes 102 have threaded surface 104 that engages and advances them into the annular tissue by rotation and preferably, the electrodes 102 may be advanced using a pushing motion (e.g., a push rod or push wire). The electrodes 102 may have an electrically conductive non-stick coating 106 so that they can be easily retracted from the tissue after heating the tissue. The electrodes 102 may be relatively stiff so that they resist bending when an approximating force is applied to pull the two electrodes together.

The first and second electrodes 102a and 102b can be contained in individual guide tubes 108a and 108b, respectively, and the catheter 100 can further include an approximating mechanism 1 10 which can pull the guide tubes 108a-b together. For example, the approximating mechanism can draw the guide tubes 108a- b together (i.e., approximate the guide tubes 108a-b) with sufficient force to overcome the naturally occurring tension in the tissue. In some embodiments, the approximating mechanism 1 10 includes a pull-wire 111W that extends through the catheter and a hinge 112 proximal of the distal tip as shown in FIG. 1. These embodiments produce an arcuate approximating motion between the first and second electrodes 102a and 102b (Indicated by arrows AA). in some embodiments, as shown in FIG. 2, the approximating mechanism 110 is connected by a linkage 114 configured to produce a linear approximating motion (indicated by arrows A_{L}) between the first and second electrodes 102a and 102b to maintain a constant orientation between the first and second electrodes 102a and 102b as they are approximated. For example, the approximating mechanism 110 in FIG. 2 can maintain a parallel relationship between the first and second electrodes 102a and 102b throughout the operational portion of the approximating motion. In some embodiments, such as shown in FIG. 2, the approximating mechanism 110 is a threaded mechanism 111S having a worm gear (not illustrated), or the like. The embodiments illustrated in FIG. 2, however, can substitute the pull-wire 111 W for the threaded mechanism 111S to operate the linkage 114.

FIG. 3 illustrates the device 100 in which the approximating mechanism 110 includes a contraction member 116 around the first and second electrodes 102a-b and an expansion member 118 interposed between first and second electrodes 102a-b. The contraction member 116 pulls the two electrodes 102a-b together (approximated), while the expansion member 118 drives the electrodes 102a-b apart from each other. In some embodiments, the contraction member 116 is an elastic sleeve and the expansion member 118 is a balloon 118 or the like. The expansion member 118 is configured to overcome the biasing force of the contraction member 116 for driving the electrodes 102a-b apart from each other. For example, when the expansion member 118 is a balloon, inflating the balloon with a fluid such as saline or the like will overcome the approximation force of the contraction member 116 and thereby further separate the electrodes 102a-b from each other. Deflating the balloon by withdrawing some of the fluid from the balloon allows the approximation force from the contraction member 116 to overcome the expansion force of the balloon and thereby approximate the electrodes 102. The contraction member 116 can comprise one or more biasing members such as springs, elastomeric members, a worm gear or the like interconnecting the electrodes 102a-b and/or the tubes 108a-b instead of an elastic sleeve. One of ordinary skill in the art will appreciate that many alternative mechanisms may be used to adjust the spacing between the electrodes 102.

The catheters 100 shown in FIGs. 1-3 can further include a first sensor 130a at the first electrode 102a and a second sensor 130b at the second electrode 102b (collectively "sensors 130"). The sensors 130 can be impedance sensors or thermistors embedded into one or both of the electrodes 102. The sensors 130 can monitor the temperature or impedance of the tissue to determine the status of the tissue before, during and/or after applying energy to the tissue via the electrodes 102a-b. The sensors 130 can send signals to a controller for ensuring electrode operation, ensuring electrode contact, controlling the extent of shrinkage, avoiding overtreatment, etc. For example, the signals from the sensors 130 can be used to determine the total energy delivered to the tissue based on the relative spacing of the electrodes or estimate the distance between the electrodes.

The electrodes 102a-b may be solid members (e.g., solid wires), or they may be tubes having a longitudinal lumen (e.g., hollow wires - not shown) and distal side-apertures (not shown). The lumens, for example, may extend through the full longitudinal length of the electrodes 102a-b, and the side-apertures may be in fluid communication with the lumens such that fluid introduced into the lumens exits through the apertures. A saline or hypertonic saline can be infused via the lumen and apertures while applying energy via the electrodes 102a-b to expand the effective area of heating and to control the extent of tissue desiccation at the electrodes 102a-b. Alternatively, the electrodes 102a-b can be cooled via circulation of fluid through them to prevent overheating of the electrodes while the intervening tissue is being heated.

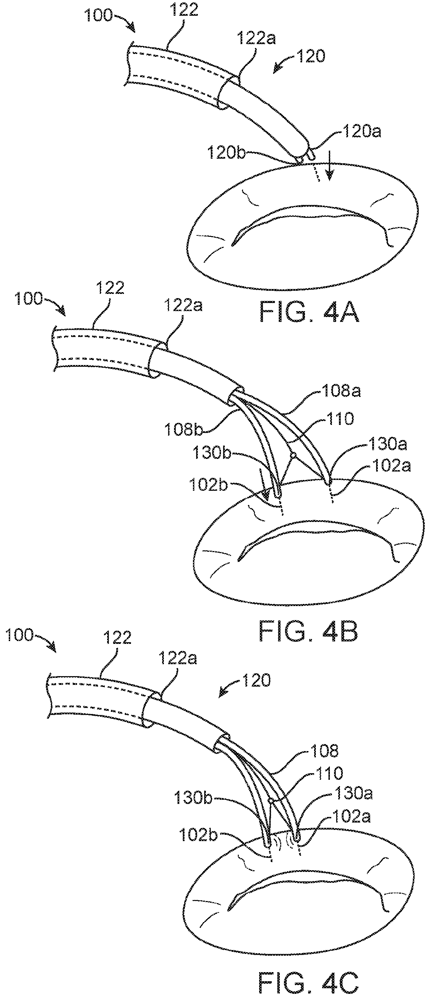

FIGs. 4A-4D illustrate an example of the operation of the device 100 shown in FIG. 1. A person skilled in the art will understand that the devices 100 shown in FIGs. 2 and 3 operate in an analogous manner. In use, the distal end of the energy delivery catheter 120 is first positioned near or against cardiac tissue such as the mitral valve annulus. (See FIG. 4A.) The energy delivery catheter 120 may be introduced to the left atrial surface of the annulus via a trans-septal or a trans-atrial approach, or it may be delivered against the ventricular surface of the annulus via a trans-aortic or a trans-apical approach. The energy catheter 120 or the guide catheter 122 may be manipulated to position the tip 120a of the catheter 120 near or in contact with appropriate annular tissue. The first electrode 102a is then advanced into the annular tissue, as shown in FIG. 4A. The first or second electrodes 102a-b can be advanced into the annular tissue independently of each other, or they can be advanced into the tissue together. The electrodes 102 are exposed by unsheathing the energy delivery catheter 120 from the guide catheter 122 or extending the energy delivery catheter 120 from the guide catheter 122, and then withdrawing the energy delivery catheter 120 with respect to the tubes 108a-b. As the energy delivery catheter 120 is withdrawn, the electrodes 102a-b can be self-biased to move further apart. The second electrode 102B can then be advanced into the tissue. (See FIG. 4B.) An approximating force is applied to pull the two electrodes 102a-b together, which cinches the annulus tissue between the electrodes 102a-b and thereby reduces the overall diameter of the annulus. (See FIG. 4C.) For example, the electrodes 102a-b might be inserted into the annular tissue spaced 10mm apart, and then pulled together to a separation of 2 mm-8 mm, or 3 mm-7 mm, or about 5 mm. The device 100 shown in FIGs. 4A-4C pulls the electrodes 102a-b together using the pull-wire 111W described above with reference to FIG. 1, but the approximating mechanism can use a worm gear, linkage or the like as described above with reference to FIGs. 2 and 3 to approximate the two electrodes.

After the electrodes 102a-b are spaced apart by a desired distance, energy is then applied between the electrodes 102a, 102b to heat the tissue for a desired time, (e.g., 15 seconds) until the collagen is adequately denatured so that the annulus retains the new smaller circumference. The energy may be bipolar RF energy, monopolar RF energy, laser energy, ultrasonic energy, resistive heating of the electrodes, microwave energy, or other energy modalities. The energy is applied based on the power and time to cause the desired amount of shrinkage without undesired disruption of the tissue. For example, the energy can be applied at 10W-100W, or 15W-85W, or 20W-70W, or 25W-55W, or 10W, 15W, 20W, 25W, 30W, 40W, 45W, 50W, 55W, 60W, 65W, 70W, 75W, 80W, 85W, 90W, 95W or 100W, or any suitable wattage therebetween. Additionally, the energy can be applied for 5s-300s, or 10s-240s, or 10s-60s, or 10s, 15s, 20s, 25s, 30s, 35s, 40s, 45s, 50s, 55s, or 60s. A chemical agent (e.g., phenol, glutaraldehyde or other fixative chemicals) may be applied to the cardiac tissue between the two electrodes in addition to or in substitution of delivering electromagnetic or mechanical energy via the first and second electrodes 102a-b.

Although bipolar RF energy has the advantage of being naturally directed between the two electrodes for heating the tissue so that it shrinks in the desired area, other energy modalities could also be applied. For example, monopolar RF energy, laser energy, ultrasonic energy, resistive heating of the electrodes, microwave energy, or other energy modalities can be used with any of the catheters 100 described above in addition to or in lieu of RF energy. Additionally, chemical methods could also be used to form the tissue into a desired shape, such as the injection of small amounts of phenol, glutaraldehyde or other fixative chemicals.

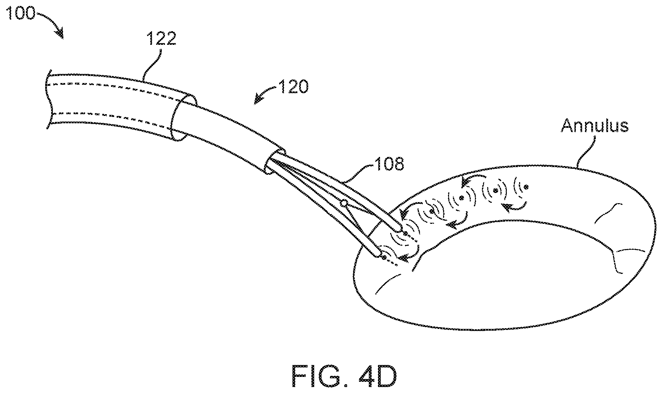

The process described above with reference to FIGs. 4A-4C can be repeated at different areas of the annulus to further reduce the circumference of the annulus in selected regions and thereby selectively reshape the annulus to promote coaptation. Referring to FIG. 4D, for example, after the tissue has been approximated, heated, shrunk, and sufficiently cooled in a first region of the annulus, at least one of the electrodes 102a-b can be withdrawn from the tissue and moved to another section of annular tissue. If it is desired to treat the adjacent tissue on one side or the other of the first region of the annulus, the first electrode 102a can be removed from the tissue while the second electrode 102b remains in the tissue, and then the energy delivery catheter 120 can be (pivoted) rotated 180 degrees around the second electrode 102b such that the first electrode 102a is on the other side of the second electrode 102b. The first electrode 102a can then be advanced into the tissue at the new location such that the first and second electrodes 102a-b span a second region of the annulus adjacent to the first region. The treatment can then continue by applying energy to the second region of the annulus via the first and second electrodes 102a-b. In this manner, the catheter can be "walked" from one region of the annulus tissue to an adjacent region while remaining attached to the annulus at all times. This is expected to make repositioning the electrodes 102a-b much faster and simpler.

In any of the foregoing embodiments, the guide catheter 122 can be used to position the energy delivery catheter 120 on or near the mitral annulus. For example, the guide catheter 120 can be inserted into the femoral vein and advanced across the interatrial septum of the heart until a tip 122a of the guide catheter 122 is positioned in the left atrium. The energy delivery catheter 120 can be inside the guide catheter 122 at this point. The guide catheter 122 can then be flexed until the tip 122a is open towards a location on the mitral annulus. The energy delivery catheter 120 can then be advanced distally through the guide catheter 122 until the electrodes 102 are at or near the mitral annulus. One or both of the electrodes 102a-b can be advanced into the annular tissue as described above with respect to Figure 4A. For example, once the first electrode 102a is fixed in position, the guide catheter 122 can be withdrawn to allow the two electrodes to move laterally apart from each other. The energy delivery catheter 120 can be rotated until the second electrode 102b is positioned over the mitral annulus, and the second electrode 102b can be advanced into the annulus as described above with respect to 4B. The two electrodes 102a-b can be pulled toward each other until they are spaced apart by a distance that places the tissue in a desired tensile state. Energy can then be delivered to the tissue via the first and second electrodes 102a-b. After a sufficient amount of energy is delivered to the tissue between the first and second electrodes 102a-b, the first electrode 102a can be removed and repositioned at an adjacent section of the annulus for sequential treatment. FIG 4D shows an example of the resulting annular shrinkage of the annulus.

Several of the foregoing embodiments can be modified to use a single electrode and/or a chemical delivery device instead of requiring two electrodes. For example, instead of having the two active electrodes 102a and 102b, the catheters 100 described above with reference to FIGs. 1-3 can have electrically inactive arms configured to extend from the guide tubes 108a-b and a monopolar electrode and/or a chemical injection needle configured to extend between the arms. In operation, the approximating mechanism 110 can draw the guide tubes 108a-b toward each other to move the electrically inactive arms closer together, as described above, and then (a) electrical energy can be applied to the tissue between the arms using the monopolar electrode and/or (b) a chemical shrinking agent can be applied to the tissue between the arms via the chemical injection needle.

This concept has been described for performing mitral annuloplasty, but it can similarly be applied to the tricuspid annulus. The elasticity of the tricuspid annulus is even more pronounced than the mitral annulus, so each segment might be compressed more before delivering energy. For example, each segment might be compressed to one-third of its pre-treatment length before delivering energy.

### Chordae Tendineae Shortening

Mitral prolapse or regurgitation may be attributable to overly long chordae tendineae. The chordae tendineae are taut and linear during systole and become limp and tortuous during diastole. It has been previously proposed to shorten chordae by applying energy to heat and shrink the chordae. Previous techniques involved placing an electrode against the chordae tendineae and applying energy until the chord shrinks appropriately. This is an uncontrolled method which may easily result in excessive shrinkage of a chord, which could end up "tethering" the leaflets and preventing closure of the valve. Moreover, it may be difficult to control the chords and to visualize how much shrinkage is occurring.

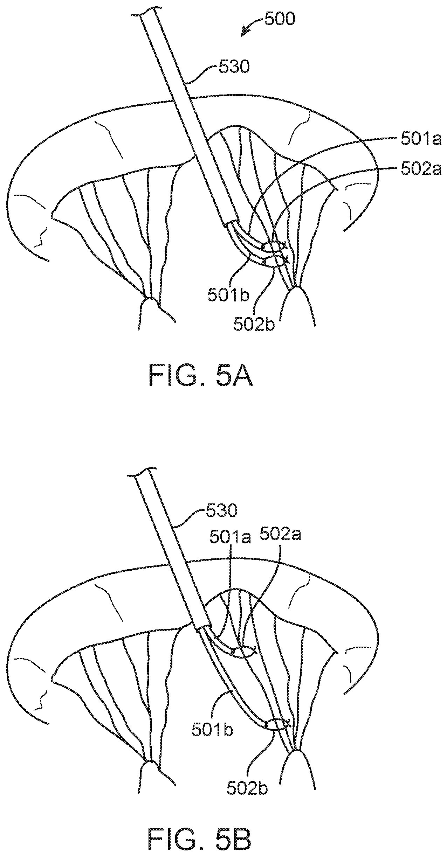

FIGs. 5A-5D show a procedure for selectively and controllably heating and shrinking the chordae tendineae using a device 500 having energy delivery mechanisms 501 (identified individually as first energy delivery mechanism 501a and second energy delivery mechanism 501b). The first and second energy delivery mechanisms 501a-b are configured to grasp one or more chordae in two places a certain distance apart. The energy delivery mechanisms 501a-b can then be approximated by the desired length of shrinkage, and energy is then delivered between the energy delivery mechanisms 501a-b to shrink the portion of the chordae between the energy delivery mechanisms 501a-b. For example, the first energy delivery mechanism 501a could be biased at one polarity and the second energy delivery mechanism 501b could be biased at the opposite polarity such that the current flows through the region of the chordae between the first and second energy delivery mechanisms 501a-b.

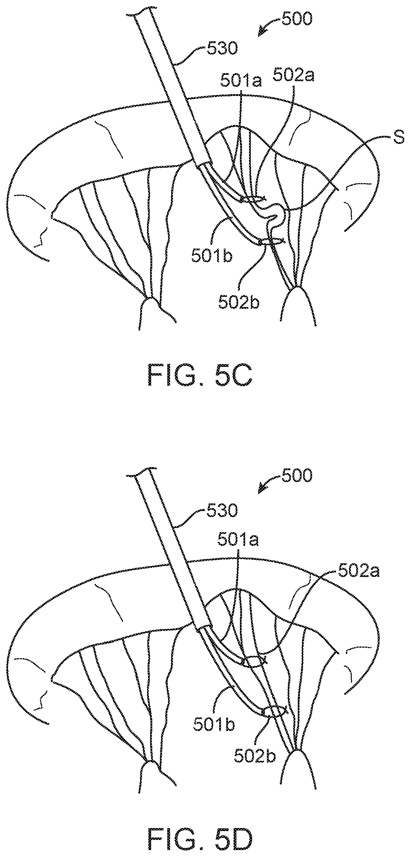

Grasping a chord or group of chords in a beating heart may be challenging. For example, it may be hard to maneuver existing catheter-based systems to grasp the same chord such that the electrodes are spaced apart by a desired distance. One solution to this challenge is shown in FIGs. 5A-5D. Referring to FIG. 5A, the first and second energy delivery mechanisms 501a-b are initially close together, possibly at an oblique angle to the axis of the catheter to minimize their cross-sectional profile for delivery through a guide catheter 530. The energy delivery elements 501a-b can have jaws 502a-b, respectively, configured to be: (a) open for receiving a chord; (b) partially closed to retain the chord while being able to slide along the chord; and (c) fully closed to grasp the chord to prevent the chord from sliding with respect to the jaws 502a-b. Referring to FIGs. 5A and 5B together, the first and second energy delivery mechanisms 501a-b can be placed near each other at first region of a chord (FIG. 5A), and then the first energy delivery mechanism 501a can be moved apart from the second energy delivery mechanism 501b to space the first and second energy delivery mechanisms 501a-b apart from each other along the chord (FIG. 5B). The first and second jaws 502a-b can then be firmly clamped against the chord and the moved closer together (approximated) such that a certain amount of slack S is induced in the chord, as shown in FIG. 5C. Energy can then be applied between the first and second energy delivery mechanisms 501a-b to preferentially and controllably shrink the chord in the longitudinal direction of the chord, as shown in FIG. 5D. After the chord has achieved a desired length, the jaws 502a-b can be released (e.g., opened) to release the chord. Valve performance can then be re-assessed and, if needed, energy can be reapplied to further shrink the chord or other chords can be shrunk.

The device 500 can be placed at the chords using a trans-apical, trans-aortic, trans-atrial, or trans-septal approach. In this setting, ultrasonic imaging, especially 3-dimensional trans-esophageal imaging, will be very helpful in managing the procedure. This device could also be used in a surgical setting, with visual confirmation of the chord grasping and length to be shortened.

The energy may be bipolar RF energy applied between the first and second jaws 502a-b, monopolar RF energy, laser energy, ultrasonic energy, resistive heating of the electrodes, microwave energy, or other energy modalities. Bipolar energy may have the advantage of directing energy to the tissue between the two jaws. A chemical agent (e.g., phenol, glutaraldehyde or other fixative chemicals) may be applied to the cardiac tissue between the two jaws 502a-b in addition to or in substitution of the energy delivery.

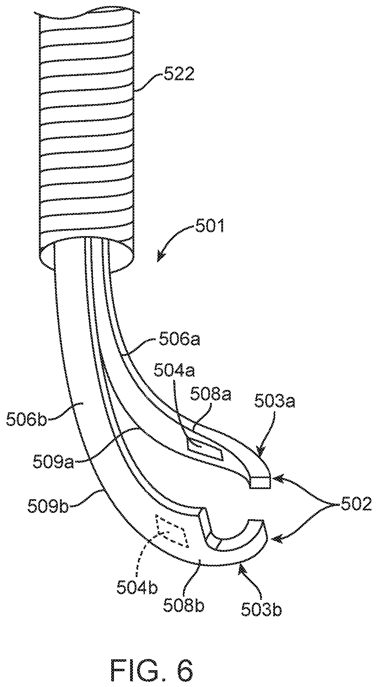

FIG. 6 illustrates some embodiments of the energy delivery mechanism 501 of the device 500 described above with reference to FIGs. 5A-5D. In some embodiments, the energy delivery mechanism 501 has a jaw 502 with a first jaw portion 503a and a second jaw portion 503b, and the first and second jaw portions 503a-b include first and second electrical contacts 504a-b, respectively, (identified collectively as "contacts 504"). Each of the first and second jaw portions 503a-b can have a shaft 506a-b, respectively, and a grasping portion 508a-b, respectively. The shafts 506a-b are configured to extend longitudinally along the length of the device and be manipulated to move the grasping portions 508a-b toward/away from each other. The shafts 506a-b and grasping portions 508a-b can be electrically conductive and coated with a dielectric material except for the areas of the contacts 504a-b. Alternatively, the shafts 506a-b and grasping portions 508a-b can be made from a dielectric material with separate electrically conductive contacts 504a-b and wires in or on the shafts 506a-b. The energy delivery mechanism 501 can further include a coiled sleeve 522 through which the shafts 506a-b and grasping portions 508a-b can extend. In operation, the grasping portions 508a-b can be closed (e.g., clamped together) by sliding advancing the coiled sleeve 522 distally toward the grasping portions 508a-b or opened (e.g., moved apart) by sliding (retracting) the coiled sleeve proximally away from the grasping portions 508a-b. The grasping portions 508a-b can accordingly extend from the shafts 506a-b along a smooth bend 509a-b, respectively, to facilitate the closing and opening of the grasping portions 508a-b via movement of the coiled sleeve 522. The energy delivery mechanism may have only one of the electrical contacts 504a-b in some embodiments.

In operation, a common polarity can be applied to both contacts 504a-b in a single jaw 502 of one energy delivery mechanism 501. As such, two energy delivery mechanisms 501 can be used as described above with respect to FIGs. 5A-5D to apply bipolar RF energy through a chord. Or, a common electrode can be used instead of one of the energy delivery mechanisms 501a-b. Additionally, the contacts 504a-b of a single energy delivery element 501 may by biased at opposite polarities to focus the energy in the region of a chord between the contacts 504a-b.

### Leaflet Re-Shaping

Mitral valve regurgitation often happens because there is excess loose tissue in the posterior leaflet. Dr. Dwight McGoon of the Mayo Clinic developed a technique of excising a V-shaped section of the P2 section of the posterior leaflet free edge and sewing the cut edges together. More recently, surgeons have simply folded the excess tissue into the ventricle and sewed the edges of that section together without cutting the leaflets, a technique sometimes called a "foldoplasty" or "dunkoplasty." Several attempts have been made to use RF energy to shrink the leaflets, but the existing techniques do not provide appropriate control of the directionality of the shrinkage. For most patients with mitral prolapse due to excessive posterior leaflet tissue, it is desired to shrink the leaflet along the lateral-medial direction of its free edge, but not in the direction from the edge to the annulus (anterior-posterior). The present technology provides a mechanism to prevent shrinkage in the anterior-posterior direction, while encouraging shrinkage in the lateral-medial direction. Moreover, RF energy may modify the elastic modulus of the leaflet (e.g., make it stiffer) in a manner that may reduce the amount of prolapse.

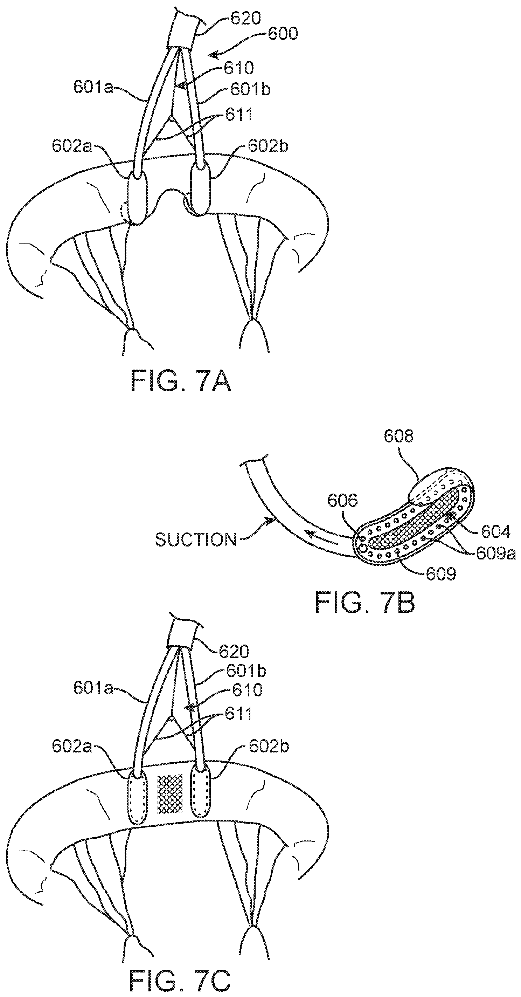

FIGs. 7A-7C show a device 600 for controlled shrinkage of leaflets via application of energy and/or through the application of a chemical agent. As shown in FIG. 7A, the device 600 includes a catheter 620 that can be introduced into the left atrium and two energy delivery arms 601 (identified individually as first and second arms 601a and 601b) having energy delivery elements 602 (identified individually as first and second energy delivery elements 602a and 602b). The energy delivery elements 602a-b can be configured to be pressed against a native leaflet of a heart valve, such as the posterior leaflet of a mitral valve, and each of the energy delivery elements 602a-b can include an electrode 604 and an aperture 606. The energy delivery elements 602a-b can be individually secured against the leaflet with suction transmitted through aperture 606. The energy delivery elements 602a-b can optionally include an extension 608 configured to wrap over the free edge of the leaflet and press the leaflet against the energy delivery element 602. The electrodes 604 can be flexible, such as an electrically conductive mesh, so that they can be securely held against the leaflet. (See, FIG. 7B.). The electrodes 604 can alternatively be a more rigid electrically conductive element. The energy delivery elements 602 may further include a face 609 having surface features 609a such as roughness, serrations, small spikes, or the like which engage the tissue and prevent it from shrinking along the length of the electrode 604 while energy is delivered, as shown in FIG. 7B. The device 600 can further include an approximating mechanism 610 having a pull-wire system 611 designed to pull the two arms 601 together before applying energy, or to freely allow the arms 601 to move closer together as the tissue shrinks. The approximating mechanism can alternatively be any of the approximating mechanisms 110 described above with reference to FIGs. 2 and 3. The energy may be bipolar RF energy, monopolar RF energy, laser energy, ultrasonic energy, resistive heating of the electrodes, microwave energy, or other energy modalities. A chemical agent (e.g., phenol, glutaraldehyde or other fixative chemicals) may be applied to the cardiac tissue between the two energy delivery elements 602 in addition to or in substitution for the energy delivery. (See, FIG. 7C.)

### Surgical Applications of these Concepts

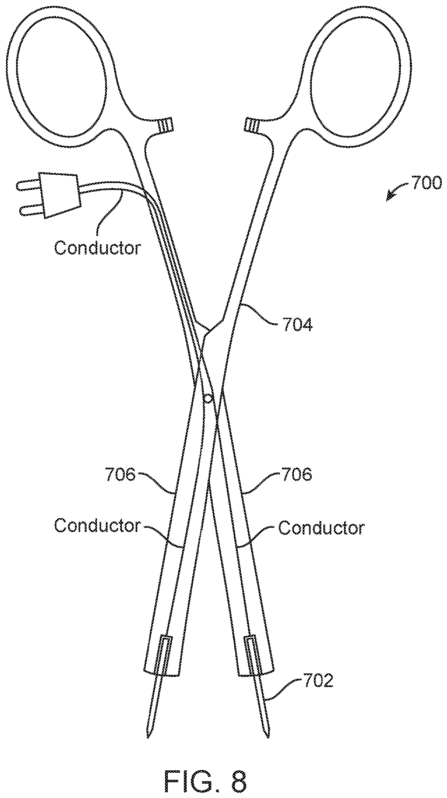

The annuloplasty, chordal shortening, and leaflet re-shaping techniques described above in accordance with the present technology can also be applied to open surgical and minimally-invasive surgical techniques. For example, FIG. 8 shows a device 700 having a pair of surgical forceps with pointed electrodes 702 on the tips which the surgeon can insert into the annular tissue. The electrodes 702 are used to approximate the tissue and to deliver energy. Electrodes 702 are electrically isolated from the forceps body 704 so that energy can be delivered between the electrodes 702. Alternatively, the electrodes 702 and arms 706 can be attached to a catheter or single-shafted instrument or the like (not illustrated), perhaps with a covering sleeve. This allows insertion through a thoracoscopic port for "Port-Access" surgery, and/or insertion through a purse-string incision in the wall of the left atrium for beating-heart surgery. The catheter or instrument shaft may be designed to be flushed to prevent the introduction of air into the bloodstream, and to prevent the backflow of blood out of the device. In some embodiments, the catheter may have an overall shaft diameter of 3-10 mm, and the shaft might be made flexible to accommodate varying surgical angles. The catheter can also be a disposable device or a reusable device. Similarly, the other concepts described above could be adapted to use in the surgical setting. The energy may be bipolar RF energy, monopolar RF energy, laser energy, ultrasonic energy, resistive heating of the electrodes, microwave energy, or other energy modalities. A chemical agent (e.g., phenol, glutaraldehyde or other fixative chemicals) may be applied to the cardiac tissue between the two electrodes in addition to or in substitution for the energy delivery.

### Combination of These Concepts with Other Technologies

It should be noted that in performing mitral valve repair, it is often desirable to perform several different repair techniques in the same procedure. For example, the cardiac tissue shrinkage techniques described in this disclosure could be combined with a chordal shrinking procedures, an edge-to-edge repair with a MitraClip^{®} device (Abbott Vascular) or other device, or other procedures.

## Claims

1. A minimally invasive device (100; 500) for reducing the dimension of a cardiac valve annulus in a beating heart, comprising:
an elongate delivery catheter (120);
at least two engagement members (102a, 102b) carried by the delivery catheter (120), wherein the engagement members (102a, 102b) are moveable between a retracted position in which the engagement members (102a, 102b) are contained within the delivery catheter (120) and an extended position in which the engagement members (102a, 102b) extend beyond a distal end of the delivery catheter (120) and wherein the engagement members (102a, 102b) are configured to engage tissue of the cardiac valve annulus in the extended position;
a tissue shrinking component configured to deliver at least one of energy and/or a chemical agent between the engagement members; and
an approximation mechanism configured to apply an approximating force having a direction to the engagement members (102a, 102b) to reduce the distance between the engagement members (102a, 102b) and reducea tension of the tissue between the engagement members (102a, 102b) to induce shrinking of the cardiac tissue in the direction of the approximating force when the tissue shrinking component delivers the at least one of the energy and/or the chemical agent wherein the tissue shrinking component comprises an electrode on each of the engagement members, and wherein the electrodes are configured to pierce into the cardiac valve annulus at a distance from each other and wherein at least one electrode has a threaded surface (104) configured to advance into annular tissue by rotation.

2. The minimally invasive device (100) of claim 1, wherein the tissue shrinking component comprises an energy delivery mechanism configured to deliver an energy modality selected from the group of bipolar, resistive heating, ultrasound, laser, and microwave.

3. The minimally invasive device (100) of any of claims 1 or 2, wherein the tissue shrinking component comprises a chemical agent selected from the group of phenol and glutaraldehyde.

4. The minimally invasive device (100) of any of claims 1 to 3, wherein the approximation mechanism (110) is operably connected to the engagement members.

5. The minimally invasive device (100) of any of claims 1 to 4, wherein the approximation mechanism (110) includes a linkage (114) connecting the engagement members (102a, 102b).

6. The minimally invasive device (100) of claim 5, wherein the linkage (114) includes a hinge (112).

7. The minimally invasive device (100) of claim 5, wherein the approximation means comprises a pull-wire (111) connected to linkage (114) such that pulling on the pull-wire (111) applies a biasing force to the engagement members (102a, 102b).

8. The minimally invasive device (100) of any of claims 1 to 7, wherein the approximation mechanism (110) includes a sleeve (116) surrounding at least a portion of the engagement members (102a, 102b) wherein advancing the sleeve (116) biases the engagement members (102a, 102b) together.

9. The minimally invasive device (100) of any one of the preceding claims, wherein the electrodes are configured to be inserted into annular tissue using a pushing motion.

10. The minimally invasive device (100) of any one of claims 1, 2 or 4 to 8, wherein the tissue shrinking component comprises an energy delivery mechanism configured to apply energy to chordae tendinea in a direction of the approximating force.

11. The minimally invasive device (500) of any one of claims 1 to 8, wherein the tissue shrinking component comprises an energy delivery mechanism (501) having a jaw with a first jaw portion (502a) and a second jaw portion (502b), the first jaw portion (501a) including a first electrical contact and the second jaw portion (502b) including a second electrical contact.

12. The minimally invasive device (100) of any of claims 1 to 11, wherein the tissue shrinking component comprises an energy delivery mechanism having two electrodes and a chemical agent configured to be applied to the cardiac tissue between the two electrodes.

## Patentansprüche

1. Minimalinvasive Vorrichtung (100; 500) zum Reduzieren der Abmessung eines Herzklappenannulus in einem schlagenden Herzen, umfassend:
einen länglichen Zuführkatheter (120);
mindestens zwei Eingriffselemente (102a, 102b), die von dem Zuführkatheter (120) getragen werden, wobei die Eingriffselemente (102a, 102b) zwischen einer eingezogenen Position, in der die Eingriffselemente (102a, 102b) innerhalb des Zuführkatheters (120) enthalten sind, und einer ausgestreckten Position, in der sich die Eingriffselemente (102a, 102b) über ein distales Ende des Zuführkatheters (120) hinaus erstrecken, beweglich sind und wobei die Eingriffselemente (102a, 102b) konfiguriert sind, um in der ausgestreckten Position in Gewebe des Herzklappenannulus einzugreifen;
eine Gewebeschrumpfungskomponente, die konfiguriert ist, um mindestens eines von Energie und/oder einem chemischen Mittel zwischen den Eingriffselementen abzugeben; und
einen Annäherungsmechanismus, der konfiguriert ist, um eine Annäherungskraft mit einer Richtung auf die Eingriffselemente (102a, 102b) anzuwenden, um den Abstand zwischen den Eingriffselementen (102a, 102b) zu verringern und eine Spannung des Gewebes zwischen den Eingriffselementen (102a, 102b) zu reduzieren, um ein Schrumpfen des Herzgewebes in der Richtung der Annäherungskraft zu induzieren, wenn die Gewebeschrumpfungskomponente das mindestens eine von der Energie und/oder dem chemischen Mittel abgibt, wobei die Gewebeschrumpfungskomponente eine Elektrode an jedem der Eingriffselemente umfasst, und wobei die Elektroden konfiguriert sind, um in den Herzklappenannulus in einem Abstand voneinander einzudringen und
wobei mindestens eine Elektrode eine Gewindeoberfläche (104) aufweist, die konfiguriert ist, um durch Rotation in ringförmiges Gewebe vorzudringen.

2. Minimalinvasive Vorrichtung (100) nach Anspruch 1, wobei die Gewebeschrumpfungskomponente einen Energieabgabemechanismus umfasst, der konfiguriert ist, um eine Energiemodalität abzugeben, die aus der Gruppe von bipolar, resistiver Erwärmung, Ultraschall, Laser und Mikrowelle ausgewählt ist.

3. Minimalinvasive Vorrichtung (100) nach einem der Ansprüche 1 oder 2, wobei die Gewebeschrumpfungskomponente ein chemisches Mittel umfasst, das aus der Gruppe von Phenol und Glutaraldehyd ausgewählt ist.

4. Minimalinvasive Vorrichtung (100) nach einem der Ansprüche 1 bis 3, wobei der Annäherungsmechanismus (110) funktionsfähig mit den Eingriffselementen verbunden ist.

5. Minimalinvasive Vorrichtung (100) nach einem der Ansprüche 1 bis 4, wobei der Annäherungsmechanismus (110) ein Gestänge (114) einschließt, das die Eingriffselemente (102a, 102b) verbindet.

6. Minimalinvasive Vorrichtung (100) nach Anspruch 5, wobei das Gestänge (114) ein Scharnier (112) einschließt.

7. Minimalinvasive Vorrichtung (100) nach Anspruch 5, wobei das Annäherungsmittel einen Zugdraht (111) umfasst, der mit dem Gestänge (114) derart verbunden ist, dass das Ziehen an dem Zugdraht (111) eine Vorspannkraft auf die Eingriffselemente (102a, 102b) ausübt.

8. Minimalinvasive Vorrichtung (100) nach einem der Ansprüche 1 bis 7, wobei der Annäherungsmechanismus (110) eine Hülse (116) einschließt, die mindestens einen Abschnitt der Eingriffselemente (102a, 102b) umgibt, wobei das Vorschieben der Hülse (116) die Eingriffselemente (102a, 102b) zusammen vorspannt.

9. Minimalinvasive Vorrichtung (100) nach einem der vorstehenden Ansprüche, wobei die Elektroden konfiguriert sind, um mittels einer Stoßbewegung in ringförmiges Gewebe eingeführt zu werden.

10. Minimalinvasive Vorrichtung (100) nach einem der Ansprüche 1, 2 oder 4 bis 8, wobei die Gewebeschrumpfungskomponente einen Energieabgabemechanismus umfasst, der konfiguriert ist, um Energie auf Chordae tendinea in einer Richtung der Annäherungskraft anzuwenden.

11. Minimalinvasive Vorrichtung (500) nach einem der Ansprüche 1 bis 8, wobei die Gewebeschrumpfungskomponente einen Energieabgabemechanismus (501) mit einer Backe mit einem ersten Backenabschnitt (502a) und einem zweiten Backenabschnitt (502b) umfasst, wobei der erste Backenabschnitt (501a) einen ersten elektrischen Kontakt einschließt und der zweite Backenabschnitt (502b) einen zweiten elektrischen Kontakt einschließt.

12. Minimalinvasive Vorrichtung (100) nach einem der Ansprüche 1 bis 11, wobei die Gewebeschrumpfungskomponente einen Energieabgabemechanismus mit zwei Elektroden und ein chemisches Mittel umfasst, das konfiguriert ist, um auf das Herzgewebe zwischen den zwei Elektroden aufgebracht zu werden.

## Revendications

1. Dispositif mini-invasif (100 ; 500) permettant de réduire la dimension d'un anneau d'une valve cardiaque dans un cœur battant, comprenant :
un cathéter d'administration allongé (120) ;
au moins deux éléments de mise en prise (102a, 102b) portés par le cathéter d'administration (120), dans lequel les éléments de mise en prise (102a, 102b) sont mobiles entre une position rétractée dans laquelle les éléments de mise en prise (102a, 102b) sont contenus à l'intérieur du cathéter d'administration (120) et une position étendue dans laquelle les éléments de mise en prise (102a, 102b) s'étendent au-delà d'une extrémité distale du cathéter d'administration (120) et dans lequel les éléments de mise en prise (102a, 102b) sont conçus pour venir en prise avec le tissu de l'anneau de la valve cardiaque dans la position étendue ;
un composant de rétrécissement de tissu conçu pour administrer au moins l'un parmi une énergie et/ou un agent chimique entre les éléments de mise en prise ; et
un mécanisme de rapprochement conçu pour appliquer une force de rapprochement ayant une direction aux éléments de mise en prise (102a, 102b) afin de réduire la distance entre les éléments de mise en prise (102a, 102b) et de réduire une tension du tissu entre les éléments de mise en prise (102a, 102b) pour induire un rétrécissement du tissu cardiaque dans la direction de la force de rapprochement lorsque le composant de rétrécissement du tissu administre au moins l'un parmi l'énergie et/ou l'agent chimique, dans lequel le composant de rétrécissement du tissu comprend une électrode sur chacun des éléments de mise en prise, et dans lequel les électrodes sont configurées pour percer l'anneau de la valve cardiaque à une certaine distance l'une de l'autre et
dans lequel au moins une électrode a une surface filetée (104) conçue pour avancer dans le tissu annulaire par rotation.

2. Dispositif mini-invasif (100) selon la revendication 1, dans lequel le composant de rétrécissement du tissu comprend un mécanisme d'administration d'énergie configuré pour administrer une modalité d'énergie choisie parmi le groupe comprenant l'énergie bipolaire, le chauffage par résistance, les ultrasons, le laser et les micro-ondes.

3. Dispositif mini-invasif (100) selon l'une quelconque des revendications 1 ou 2, dans lequel le composant de rétrécissement du tissu comprend un agent chimique choisi parmi le groupe comprenant le phénol et le glutaraldéhyde.

4. Dispositif mini-invasif (100) selon l'une quelconque des revendications 1 à 3, dans lequel le mécanisme de rapprochement (110) est relié de manière opérationnelle aux éléments de mise en prise.

5. Dispositif mini-invasif (100) selon l'une quelconque des revendications 1 à 4, dans lequel le mécanisme de rapprochement (110) comporte une liaison (114) reliant les éléments de mise en prise (102a, 102b).

6. Dispositif mini-invasif (100) selon la revendication 5, dans lequel la liaison (114) comporte une charnière (112).

7. Dispositif mini-invasif (100) selon la revendication 5, dans lequel le moyen de rapprochement comprend un fil de traction (111) relié à la liaison (114) de telle sorte que la traction sur le fil de traction (111) applique une force de sollicitation aux éléments de mise en prise (102a, 102b).

8. Dispositif mini-invasif (100) selon l'une quelconque des revendications 1 à 7, dans lequel le mécanisme de rapprochement (110) comporte un manchon (116) entourant au moins une partie des éléments de mise en prise (102a, 102b), dans lequel l'avancement du manchon (116) sollicite les éléments de mise en prise (102a, 102b) conjointement.

9. Dispositif mini-invasif (100) selon l'une quelconque des revendications précédentes, dans lequel les électrodes sont configurées pour être insérées dans le tissu annulaire par un mouvement de poussée.

10. Dispositif mini-invasif (100) selon l'une quelconque des revendications 1, 2 ou 4 à 8, dans lequel le composant de rétrécissement du tissu comprend un mécanisme d'administration d'énergie configuré pour appliquer de l'énergie au cordage tendineux dans une direction de la force de rapprochement.

11. Dispositif mini-invasif (500) selon l'une quelconque des revendications 1 à 8, dans lequel le composant de rétrécissement du tissu comprend un mécanisme d'administration d'énergie (501) ayant une mâchoire avec une première partie de mâchoire (502a) et une seconde partie de mâchoire (502b), la première partie de mâchoire (501a) comportant un premier contact électrique et la seconde partie de mâchoire (502b) comportant un second contact électrique.

12. Dispositif mini-invasif (100) selon l'une quelconque des revendications 1 à 11, dans lequel le composant de rétrécissement du tissu comprend un mécanisme d'administration d'énergie ayant deux électrodes et un agent chimique conçu pour être appliqué au tissu cardiaque entre les deux électrodes.
